# EUROPÄISCHE PATENTANMELDUNG

(11) **EP 2 529 744 A1**
(43) Veröffentlichungstag der Anmeldung: **05.12.2012**
(21) Anmeldenummer: 11004551.5
(22) Anmeldetag: 03.06.2011
(51) Int. Cl.: A61K 36/03, A61K 36/02, A61K 36/05, A61K 36/04

(54) **Proteinextrakt aus marinen Makroalgen, Verfahren zu dessen Herstellung und Verwendungen dafür**

(71) Anmelder: Fraunhofer Gesellschaft zur Förderung der angewandten Wissenschaft E.V., 80686 München (DE)
(72) Erfinder: Kruse, Charli, 23923 Herrnburg (DE); Symanowski, Frauke, 23684 Gleschendorf (DE); Gebert, Marina, 22763 Hamburg (DE)
(74) Vertreter: Katzameyer, Michael

(57) **Zusammenfassung**

Die Erfindung betrifft ein Verfahren zur Herstellung von Proteinextrakten aus marinen Makroalgen, die Proteinextrakte selbst und deren Verwendungen, insbesondere in der Medizin, Zellkulturtechnik und anderen Gebieten.

Das erfindungsgemäße Verfahren zur Herstellung eines Proteinextrakts aus Makroalgen umfasst die folgenden Schritte:
a) Bereitstellen eines zerkleinerten Algenausgangsmaterials;
b) Aufnehmen des zerkleinerten Algenausgangsmaterials in wässrigem Extraktionspuffer, der auf einen pH-Wert von 3-10 eingestellt ist;
c) Extrahieren des Materials bei Temperaturen im Bereich von 0-10 °C;
d) Zentrifugieren der Extraktionsmischung bei 5-30000 g und Gewinnen des Überstands.

Proteinextrakte, die mit dem erfindungsgemäßen Verfahren hergestellt wurden, zeigen in Abhängigkeit von dem verwendeten Algenausgangsmaterial eine proliferationsfördernde oder proliferationshemmende Wirkung auf Einzelzellen.

## Beschreibung

Die Erfindung betrifft ein Verfahren zur Herstellung von Proteinextrakten aus marinen Makroalgen, die Proteinextrakte selbst und deren Verwendungen, insbesondere in der Medizin, Zellkulturtechnik und anderen Gebieten.

Algen gehören zu den größten Biomasseproduzenten der Meere und erfüllen wichtige Funktionen im Ökosystem: Als Primärproduzenten bilden sie die Nahrungsgrundlage für viele assoziierte Arten, durch ihre photosynthetische Aktivität binden sie Kohlendioxid und entziehen dem Wasser Nährstoffe. Als bestandsbildende Arten liefern sie für viele weitere marine Organismen Habitat und Laichplatz, werden gleichzeitig auch als Siedlungsfläche von Bakterien, Bryozoen, Balaniden und anderen Aufwuchsorganismen genutzt und tragen so zur Diversität und Strukturierung der Lebensgemeinschaften bei.

Anhand der unterschiedlichen Pigmentzusammensetzung werden Makroalgen in die drei Abteilungen Grünalgen (Chlorophyta), Rotalgen (Rhodophyta) und Braunalgen (Phaeophyta) eingeteilt.

Die Vielfalt von Algen spiegelt sich auch in ihren Inhaltsstoffen wieder. Sie sind außergewöhnlich reich an Sekundärmetaboliten, deren Wirkungsspektrum sich über antibakterielle, antivirale, antifungale, antiadhäsive, entzündungshemmende und weitere Effekte erstreckt. Noch kaum untersucht sind die verschiedenen Proteine in Makroalgen und ihre möglichen biotechnologischen Anwendungen. Im Zusammenhang mit Zellkulturtechniken könnten sie hier die verwendeten tierischen Proteine aus fötalem Kälberserum (FKS) ersetzen und als Wachstumsfaktoren fungieren. Proteine, welche dagegen wachstumshemmende Eigenschaften besitzen, sind vielversprechende Kandidaten für die Tumortherapie und die Krebsforschung.

Der Erfindung liegt daher die Aufgabe zu Grunde, neue verbesserte Verfahren bereitzustellen, mit denen Proteine aus Algen, insbesondere Makroalgen, leicht, kostengünstig, schonend und effizient in guter Ausbeute gewonnen werden können. Eine damit in Zusammenhang stehende Aufgabe ist die Bereitstellung von Proteinextrakten aus Makroalgen, die vorteilhafte Eigenschaften, insbesondere für medizinische, kosmetische und andere Anwendungen, aufweisen.

Diese Aufgabe wird erfindungsgemäß gelöst durch die Bereitstellung des Verfahrens nach Anspruch 1 sowie des Proteinextrakts nach Anspruch 13. Weitere Aspekte und/oder bevorzugte Ausführungsformen der Erfindung sind Gegenstand der weiteren Ansprüche.

### Beschreibung der Erfindung

Das erfindungsgemäße Verfahren zur Herstellung von Proteinextrakten aus marinen Makroalgen umfasst mindestens die folgenden Schritte:
a) Bereitstellen eines zerkleinerten Algenausgangsmaterials;
b) Aufnehmen des zerkleinerten Algenausgangsmaterials in wässrigem Extraktionspuffer, der auf einen pH-Wert von 3-10 eingestellt ist;
c) Extrahieren des Materials bei Temperaturen im Bereich von 0-10 °C;
d) Zentrifugieren der Extraktionsmischung bei 5-30000 g und Gewinnen des Überstands.

Als Algenausgangsmaterial sind grundsätzlich alle Makroalgen, insbesondere Braunalgen, Rotalgen und Grünalgen, geeignet.

Einige spezielle, nicht-beschränkende Beispiele sind die Braunalgen *Ascophyllum nodosum, Halidrys siliquosa, Saccharina latissima, Fucus vesiculosus,* die Rotalgen *Delesseria sanguinea, Ceramium rubrum, Dumontia contorta* und die Grünalgen *Cladophora glomera, Ulva sp. und Chaetomorpha linum.*

Die Bereitstellung des Algenausgangsmaterials umfasst typischerweise einen vorbereitenden Schritt des Entfernens von Salz und Aufwuchs vom Algenausgangsmaterial, um ein von Verunreinigungen möglichst freies Ausgangsmaterial zu erhalten. In Einzelfällen könnte auf diesen Schritt jedoch auch verzichtet werden.

Die Zerkleinerung des Algenausgangsmaterials geschieht typischerweise durch Mahlen im gefrorenen oder gefriergetrockneten Zustand. Anschließend liegt das Ausgangsmaterial ganz oder überwiegend in Pulverform vor.

Im Gegensatz zu den meisten Lösungsmitteln, die bisher zur Extraktion von Makroalgen verwendet wurden, ist das erfindungsgemäß eingesetzte Extraktionsmittel ein wässriger Extraktionspuffer, der es ermöglicht, die gewünschten Proteine sehr schonend und gleichzeitig sehr effizient zu extrahieren.

Im Gegensatz zu standardmäßig für Makroalgen verwendeten Proteinisolationstechniken kann mit dem erfindungsgemäßen Verfahren aus einer geringen Menge Algen eine sehr große Menge Proteinextrakt hergestellt werden. Das erfindungsgemäße schonende Extraktionsverfahren hat auch den Vorteil, dass auf herkömmliche organische Lösungsmittel mit potentiell zellschädigenden Auswirkungen verzichtet werden kann.

Der erfindungsgemäß verwendete wässrige Extraktionspuffer kann aus Wasser allein bestehen, enthält jedoch typischerweise Wasser und einen oder mehrere Bestandteil(e) aus der Gruppe, die Mittel zur Aufhebung von Protein-Protein-Wechselwirkungen, Mittel, um die Oxidation von SH-Gruppen nativer Proteine zu verhindern, Proteinasehemmer, Detergenzien und pH-Regler umfasst.

Spezieller enthält der Extraktionspuffer mindestens ein Mittel zur Aufhebung von Protein-Protein-Wechselwirkungen, mindestens ein Mittel, um die Oxidation von SH-Gruppen der nativen Proteine zu verhindern, mindestens einen Proteinasehemmer, mindestens ein Detergenz und mindestens einen pH-Regler.

In einer noch spezielleren Ausführungsform ist das Mittel zur Aufhebung von Protein-Protein-Wechselwirkungen aus der Gruppe aus Harnstoff, Thioharnstoff, Salzen (zur Salzfällung) und Mischungen davon ausgewählt. Harnstoff, Thioharnstoff und Mischungen davon sind bevorzugt.

In einer anderen speziellen Ausführungsform ist das Mittel, um die Oxidation von SH-Gruppen der Proteine zu verhindern, aus der Gruppe aus Dithiothreitol, Dithioerythritol, β-Mercaptoethanol, strukturell verwandten Reduktionsmitteln, die SH-Gruppen enthalten, und Mischungen davon ausgewählt.

In einer anderen speziellen Ausführungsform ist das Detergenz aus der Gruppe aus CHAPS, Triton X-100 und anderen Tritonreagenzien, SDS, anderen zwitter- oder nichtionischen Detergenzien, und Mischungen davon ausgewählt.

In einer noch spezielleren Ausführungsform ist das Verfahren dadurch gekennzeichnet, dass der Extraktionspuffer 5-10 mol/l Harnstoff, 1-3 mol/l Thioharnstoff, 0,5-2 % Dithiothreitol, 0,5-1 % pH-Regler, 1-5 % Detergenz, vorzugsweise CHAPS, sowie 1-10 mmol/l Proteinasehemmer enthält. In Einzelfällen könnten jedoch durchaus auch andere Bereiche für die jeweiligen Bestandteile geeignet sein.

Ein ähnlicher Puffer ist für die Extraktion von Proteinen aus tierischen Zellen (Schneckenzellen) bekannt (Schrattenholz et al. "Methoden der Proteomforschung-Molekulare Analyse der Proteinexpression", Heidelberg, Berlin: Spektrum Akademischer Verlag 2001). In Anbetracht der Tatsache, dass die Arbeit mit pflanzlichen Zellen üblicherweise grundlegend verschieden von der mit tierischen Zellen ist, war es sehr überraschend, dass hier die Übertragung von einem Gebiet auf das andere mit großem Erfolg möglich war.

Die Extraktion erfolgt für eine Zeitspanne von 1-60 Minuten, z.B. 1-10 Minuten, unter mechanischer Bewegung bei tiefer Temperatur von 0-10°C. Im Ausführungsbeispiel ist gezeigt, dass typischerweise bereits eine sehr kurze Extraktionszeit von ca. 3 Minuten bei einer sehr niedrigen Temperatur von ca. 0°C (Eiskühlung) ausreicht, um die gewünschten Proteine in guter Ausbeute zu erhalten.

Die mechanische Bewegung kann durch Rühren und/oder Schütteln erfolgen.

Allgemein ist hier wichtig, dass die Probe ständig so gekühlt wird, dass eine Degradation der Proteine verhindert wird.

Anschließend werden die festen Bestandteile durch Zentrifugation von der Probe abgetrennt. Die angewandte Zentrifugalkraft und -dauer muss stark und lange genug sein, um Zelldebris zu entfernen, darf aber nicht so hoch sein, dass die Proteine sedimentieren. Geeignete Bedingungen im Rahmen der oben genannten Bereiche können vom Fachmann unschwer durch Routineversuche ermittelt werden.

Gewünschtenfalls kann der Proteinextrakt noch mit einem bekannten Verfahren, z.B. mittels im Handel erhältlicher geeigneter Zentrifugationssäulen, von Detergenz befreit werden (optionaler zusätzlicher Schritt).

Die mit dem erfindungsgemäßen Verfahren erhaltenen Proteinextrakte aus Makroalgen, insbesondere Braun-, Rot- oder Grünalgen, ermöglichen eine externe Beeinflussung von Einzelzellen, vorzugsweise eukaryontischen Einzelzellen, insbesondere heterotrophen Zellen. Diese eukaryontischen Zellen sind beispielsweise Zellen von Vertebraten, spezieller Säuger, einschließlich Menschen. Die Zellen können Stammzellen, Vorläuferzellen oder differenzierte Zellen sein.

Konkreter haben die dem erfindungsgemäßen Verfahren erhaltenen Proteinextrakte aus Makroalgen, insbesondere Braun-, Rotoder Grünalgen, eine proliferations- bzw. wachstumshemmende oder proliferations- bzw. wachstumsfördernde Wirkung auf die obengenannten Einzelzellen.

Untersuchungen der Erfinder haben ergeben, dass Proteinextrakte aus Braunalgen, insbesondere den Spezies *Ascophyllum nodosum, Halidrys siliquosa, Saccharina latissima, Fucus vesiculosus,* oder aus der Grünalge *Cladophora glomerata* eine proliferationshemmende Wirkung auf Einzelzellen ausüben (siehe Tab. 1).

Weitere Untersuchungen der Erfinder haben ergeben, dass Proteinextrakte aus Grünalgen, insbesondere der Grünalge *Ulva sp.*, eine proliferationsfördernde Wirkung auf Einzelzellen ausüben (siehe Tab. 1).

Der Einsatz von wachstumsfördernden Proteinen pflanzlicher Herkunft bietet großes Potential für die Kultivierung von Zellen, z.B. wenn in Bioreaktoren viel Biomasse produziert werden soll. Im Gegensatz zum fetalen Kälberserum (FKS) besteht nicht die Gefahr einer Kontamination mit Prionen, so dass die gewonnenen Zellen in der Pharmaindustrie genutzt werden können. Wachstumshemmende Proteinextrakte könnten in der Krebstherapie Verwendung finden oder zur Oberflächenbeschichtung von Implantaten herangezogen werden.

Gegenstand der vorliegenden Erfindung ist daher auch die Verwendung der Proteinextrakte für medizinische, kosmetische oder andere Anwendungen, beispielsweise in der Zellkulturtechnik zur Desinfektion von Oberflächen oder als Ersatz von tierischem Serum, oder als Anti-Fouling-Mittel bzw. Biofilminhibitor. Die Proteinextrakte können auch zur Oberflächenbeschichtung von Implantaten, Zellkulturmaterialien oder anderen Oberflächen auf denen Zellwachstum gefördert oder unterdrückt werden soll, eingesetzt werden.

### FIGURENBESCHREIBUNG

**Fig. 1** zeigt ein Balkendiagramm der proliferationshemmenden oder -fördernden Wirkung verschiedener Proteinextrakte auf Rattenzellen
**Fig. 2** zeigt die proliferationsfördernde Wirkung eines Extrakts der Grünalge *Ulva sp.* Auf die Rattenzellkultur RApan 5bP8 (links Kontrolle; rechts nach Behandlung mit Extrakt)

Die folgenden Beispiele sollen die Erfindung näher erläutern ohne diese jedoch darauf zu beschränken.

### BEISPIEL 1

### Gewinnung von Proteinextrakten aus verschiedenen marinen Makroalgen

Allgemein kann man alle Makroalgen für diese Methode verwenden. Beispielhaft werden hier nur einige Arten aufgeführt.

Die Grünalgen *Cladophora glomerata* und *Ulva* sp. wurden im Flachwasserbereich am Brodtener Ufer (53.992313 N, 10.866373 E) und die Braunalge *Saccharina latissima* am Standort Travemünde (53.973513 N, 10.883968 E) gesammelt. Ferner wurde *Fucus vesiculosus* am Standort Schilksee (54.424588, 10.174870 E), *Halidrys siliquosa* vor Helgoland (54.169259 N, 7.895154 E) und *Ascophyllum nodosum* im Kattegat nähe Saltö (58.862002 N, 11.101484 E) gesammelt. Die Proben wurden unter Kühlung in das Labor transportiert. Dort wurden sie erst mit Meerwasser, dann kurz mit Aqua dest. gespült, um eventuelle Salzrückstände sowie Epibionten zu entfernen.

Um ein Zelllysat zu erhalten, wurde das Gewebe mechanisch aufgeschlossen. Die Braunalgen *Ascophyllum nodosum, Halidrys siliquosa* und *Fucus vesiculosus* wurden gefriergetrocknet und mittels einer Labormühle gemahlen, während die übrigen Proben frisch verarbeitet wurden. Diese wurden in einen mit flüssigem Stickstoff vorgekühlten Mörser gegeben und bis zur Pulverform zerkleinert. Anschließend wurde 0,5 ml Probe in ein Eppendorfgefäß gegeben und mit 1,5 ml Extraktionspuffer versetzt. Der in diesem Beispiel verwendete Extraktionspuffer enthält 7 mol/l Harnstoff, 2 mol/l Thioharnstoff, 1% Dithiothreitol, 2 % ChAPS (Bio-Rad) 0,8 % Biolyte pH 3-10 sowie 5mmol/l Pefablock ad 10 ml A. dest.

Prinzipiell kann jedoch auch ein anderer wässriger Extraktionspuffer verwendet werden, vorzugsweise ein Puffer, der eine oder mehrere, besonders bevorzugt alle, der nachfolgend aufgeführten Komponenten enthält. Proteinaseinhibitoren (z.B. Pefablock) werden zugegeben, um proteinabbauende enzymatische Aktivitäten zu unterdrücken. Um die Löslichkeit der Proteine zu gewährleisten, werden Detergenzien wie z.B. das zwitterionische CHAPS zugesetzt, chaotrophe Substanzen wie Harnstoff und Thioharnstoff sollen Protein-Protein-Wechselwirkungen lösen. Reduzierende Verhältnisse erhält man durch die Zugabe von DTT oder anderen Reduktionsmitteln, womit die Disulfidbrücken zwischen den Polypeptidketten gelöst werden bzw. die Bildung unerwünschter Disulfidbrücken vermieden wird.

Die Probe wurde in diesem Beispiel unter ständigem Rühren für 3 min auf Eis gekühlt und anschließend bei 4°C und 13000 rpm (Modell Labofuge 400R, Festwinkelrotor für Mikrozentrifugenröhrchen 75003325 Firma Hereaus) für 3 min zentrifugiert. Allgemein ist hier wichtig, dass die Probe ständig so gekühlt wird, dass eine Degradation der Proteine verhindert wird. Die angewandte Zentrifugalkraft und -dauer muss stark und lange genug sein, um Zelldebris zu entfernen, darf aber nicht so hoch sein, dass die Proteine sedimentieren.

Der Überstand wurde abgenommen und aliquotiert. Das hier verwendete Detergenz wurde mittels Pierce^{®} Detergent Removal Spin Columns entfernt, anschließend der Proteingehalt mit der Bradfordmethode photometrisch bestimmt. Das Detergenz kann aber auch mit einer beliebigen anderen Methode von der Probe abgetrennt werden.

### BEISPIEL 2

### Wirkung der Proteinextrakte auf Rattenzellen

Beispielhaft wird hier die Untersuchung der Effekte auf die Langzeit-Zellkultur RApan5bP8 beschrieben.
Zellen der Langzeit-Zellkultur RApan5bP8 (*Rattus norvegicus)* wurden aufgetaut und in Dulbecco's Modified Eagle's Medium (DMEM) unter Zusatz von 10% FKS kultiviert. Die Zelldichte wurde am Nucleocounter ChemoMetec^{®} bestimmt. Für den Versuch wurden Multiwell Kulturplatten im 6er Format verwendet. Je well wurden 65970 Zellen ausgesät. Die Extrakte wurden in 3 Replikaten in einer Konzentration von 0,05 µg/µl getestet. Zuerst wurde der Extrakt in die Mulde ("well") pipettiert, danach die Zellsuspension im entsprechenden Volumen. Als Kontrolle diente die Zellsuspension ohne Extrakt. Die Zellen wurden für 24 h bei 37°C und 5% CO₂ im Brutschrank kultiviert, dann erfolgte die Wachstumskontrolle unter dem Mikroskop. Mittels Nucleocounter wurde die Anzahl der lebenden und toten Zellen bestimmt.

### Ergebnisse

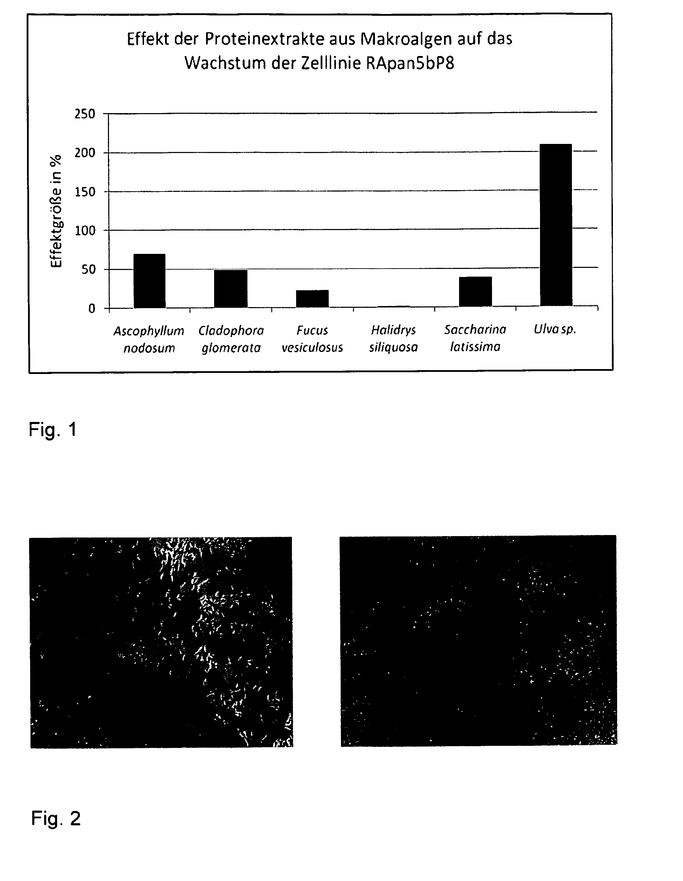

Eine wachstumshemmende Wirkung zeigten die Proteinextrakte der Braunalgen *Ascophyllum nodosum, Saccharina latissima, Fucus vesiculosus* und *Halidrys siliquosa.* Ebenfalls wachstumshemmend zeigte sich der Proteinextrakt der Grünalge *Cladophora glomerata,* wohingegen sich der Extrakt der Grünalge *Ulva* sp. als deutlich wachstumsfördernd erwies (Tab. 1; Fig. 1 und 2)

**Tab.1: Effekt der Proteinextrakte auf das Zellwachstum der Zellkultur RApan5bP8**

| Makroalge | Zellzahl Extrakt | Zellzahl Kontrolle | Effekt in % |
|---|---|---|---|
| *Ascophyllum nodosum* | 247.500,00 | 355.500,00 | 69,62 |
| *Cladophora glomerata* | 82.500,00 | 169.500,00 | 48,67 |
| *Fucus vesiculosus* | 232.500,00 | 1.039.500,00 | 22,37 |
| *Halidrys siliquosa* | 3.000,00 | 204.000,00 | 1,47 |
| *Saccharina latissima* | 303.000,00 | 777.000,00 | 39,00 |
| *Ulva sp.* | 610.500,00 | 292.500,00 | 208,72 |

### Literatur

Schrattenholz et al. "Methoden der Proteomforschung-Molekulare Analyse der Proteinexpression", Heidelberg, Berlin: Spektrum Akademischer Verlag 2001
Ginnae Ahn, Eunjin Park, Won-Woo Lee,Jin-Won Hyun, Ki-Wan Lee, Taekyun Shin,You-Jin Jeon, Youngheun Jee (2010). "Enzymatic Extract from Ecklonia cava Induces the Activation of Lymphocytes by IL-2 Production Through the Classical NF-κB Pathway". Mar Biotechnol, DOI 10.1007/s10126-010-9270-6
Elisabete Barbarino und Sergio O. Louren (2005). "An evaluation of methods for extraction and quantification of protein from marine macro- and microalgae". Journal of Applied Phycology 17: 447-460
Joel Fleurencel, Catherine Le Coeur, Serge Mabeau, Manuelle Maurice & Annie Landrein (1995). "Comparison of different extractive procedures for proteins from the edible seaweeds Ulva rigida and Ulva rotundata". Journal of Applied Phycology 7: 577-582
Eun-Young Kim, Dong-Gyun Kim, Yu-Ri Kim, Hye-Jung Hwang, Taek-Jeong Nam, In-Soo Kong (2010). "An improved method of protein isolation and proteomeanalysis with Saccharina japonica (Laminariales) incubated under different pH conditions". J Appl Phycol. DOI 10.1007/s10811-010-9550-6

## Patentansprüche

1. Verfahren zur Herstellung eines Proteinextrakts aus Makroalgen, umfassend die folgenden Schritte:
a) Bereitstellen eines zerkleinerten Algenausgangsmaterials;
b) Aufnehmen des zerkleinerten Algenausgangsmaterials in wässrigem Extraktionspuffer, der auf einen pH-Wert von 3-10 eingestellt ist;
c) Extrahieren des Materials bei Temperaturen im Bereich von 0-10 °C;
d) Zentrifugieren der Extraktionsmischung bei 5-30000 g und Gewinnen des Überstands.

2. Verfahren nach Anspruch 1, **dadurch gekennzeichnet, dass** die Bereitstellung des Algenausgangsmaterials einen Schritt des Entfernens von Salz und Aufwuchs vom Algenausgangsmaterial umfasst.

3. Verfahren nach Anspruch 1 oder 2, **dadurch gekennzeichnet, dass** der Extraktionspuffer einen oder mehrere Bestandteil(e) aus der Gruppe, die Mittel zur Aufhebung von Protein-Protein-Wechselwirkungen, Mittel, um die Oxidation von SH-Gruppen der nativen Proteine zu verhindern, Proteinasehemmer, Detergenzien und pH-Regler umfasst, enthält.

4. Verfahren nach Anspruch 3, **dadurch gekennzeichnet, dass** der Extraktionspuffer mindestens ein Mittel zur Aufhebung von Protein-Protein-Wechselwirkungen, mindestens ein Mittel, um die Oxidation von SH-Gruppen der nativen Proteine zu verhindern, mindestens einen Proteinasehemmer, mindestens ein Detergenz und mindestens einen pH-Regler umfasst.

5. Verfahren nach Anspruch 3 oder 4, **dadurch gekennzeichnet, dass** das Mittel zur Aufhebung von Protein-Protein-Wechselwirkungen aus der Gruppe aus Harnstoff, Thioharnstoff, Salzen und Mischungen davon ausgewählt ist.

6. Verfahren nach Anspruch 3 oder 4, **dadurch gekennzeichnet, dass** das Mittel, um die Oxidation von SH-Gruppen der Proteine zu verhindern, aus der Gruppe aus Dithiothreitol, Dithioerythritol, β-Mercaptoethanol, strukturell verwandten Reduktionsmitteln, die SH-Gruppen enthalten, und Mischungen davon ausgewählt ist.

7. Verfahren nach einem der Ansprüche 3 bis 6, **dadurch gekennzeichnet, dass** das Detergenz aus der Gruppe aus CHAPS, Triton X-100 und anderen Tritonreagenzien, SDS, anderen zwitter- oder nichtionischen Detergenzien, und Mischungen davon ausgewählt ist.

8. Verfahren nach einem der Ansprüche 3-7, **dadurch gekennzeichnet, dass** der Extraktionspuffer 5-10 mol/l Harnstoff, 1-3 mol/l Thioharnstoff, 0,5-2 % Dithiothreitol, 0,5-1 % pH-Regler, 1-5 % Detergenz, vorzugsweise CHAPS, sowie 1-10 mmol/l Proteinasehemmer enthält.

9. Verfahren nach einem der Ansprüche 1-8, **dadurch gekennzeichnet, dass** die Extraktion für eine Zeitspanne von 1-60 Minuten, z.B. 1-10 Minuten, unter mechanischer Bewegung erfolgt.

10. Verfahren nach einem der Ansprüche 1-9, **dadurch gekennzeichnet, dass** das Algenausgangsmaterial aus Braunalgen, Rotalgen und Grünalgen ausgewählt ist.

11. Verfahren nach Anspruch 10, **dadurch gekennzeichnet, dass** die Algen die Braunalgen *Ascophyllum nodosum, Halidrys siliquosa, Saccharina latissima, Fucus vesiculosus,* die Rotalgen *Delesseria sanguinea, Ceramium rubrum, Dumontia contorta* und die Grünalgen *Cladophora glomera, Ulva sp.* und *Chaetomorpha linum* umfassen.

12. Verfahren nach einem der Ansprüche 1-11, **dadurch gekennzeichnet, dass** das zerkleinerte Algenausgangsmaterial zum größten Teil in Pulverform vorliegt.

13. Proteinextrakt aus Makroalgen, insbesondere Braun-, Rotoder Grünalgen, erhalten mit dem Verfahren nach einem der Ansprüche 1-12, der eine proliferationsbeeinflussende Wirkung auf Einzelzellen, insbesondere eukaryontische heterotrophe Zellen, ausübt.

14. Proteinextrakt nach Anspruch 13 aus Braunalgen, insbesondere den Spezies *Ascophyllum nodosum, Halidrys siliquosa, Saccharina latissima, Fucus vesiculosus,* oder der Grünalge *Cladophora glomerata,* der eine proliferationshemmende Wirkung ausübt.

15. Proteinextrakt nach Anspruch 13 aus Grünalgen, insbesondere der Grünalge *Ulva sp.,* der eine proliferationsfördernde Wirkung ausübt.

16. Verwendung des Proteinextrakts nach einem der Ansprüche 13-15 für medizinische oder kosmetische Anwendungen, in der Zellkulturtechnik zur Desinfektion von Oberflächen oder als Ersatz von tierischem Serum, oder als Anti-Fouling-Mittel, oder als Biofilminhibitor.

17. Verwendung nach Anspruch 16 zur Oberflächenbeschichtung von Implantaten, Zellkulturmaterialien oder anderen Oberflächen auf denen Zellwachstum gefördert oder unterdrückt werden soll.
